# EUROPEAN PATENT SPECIFICATION

(11) **EP 1 632 218 B1**
(45) Date of publication and mention of the grant of the patent: **09.05.2007**
(21) Application number: 04012175.8
(22) Date of filing: 22.05.2004
(51) Int. Cl.: A61K 8/49, A61K 8/46, A61Q 5/10

(54) **Composition for dyeing keratin fibres**
Zusammensetzung zum Färben von Keratinfasern
Composition de teinture de fibres kératiniques

(43) Date of publication of application: 08.03.2006
(73) Proprietor: KPSS-Kao Professional Salon Services GmbH, 64297 Darmstadt (DE)
(72) Inventor: Nöcker, Bernd, Dr., 64372 Ober-Ramstadt (DE); Wood, Jonathan, Dr., 69469 Weinheim (DE); Kufner, Frank, 64297 Darmstadt (DE)

(56) References cited:
- EP-A- 1 166 752
- WO-A-03/022232
- WO-A-03/076518

## Description

This invention relates to a dyeing composition for keratin fibers, especially hair, comprising at least one anionic and at least one cationic direct acting hair dyes and showing excellent dyeing ability and excellent resistance to hair washing and environmental influences. The colouring composition of this invention are ready to use colouring composition and, therefore, do not require any mixing prior to application with additional agents such as oxidizers.

In another way of carrying out the invention and in the case that a brightening and lightening, in hair colour, are wished, the compositions of the present invention can as well be used after mixing with an oxidizing agent.

Hair colouring is a common practice for ages. Oxidative colouration has widely been used for achieving durable, brilliant hair colour. Direct dyes, mainly of cationic character, have also found their applications for colouring hair. Recently, anionic direct dyes have as well been found to be very powerful for changing hair colour permanently and to achieve long lasting, brilliant colours in strong acidic medium. The colouring agents with anionic dyes are so formulated that the optimum conditions are realised for achieving the highest dyestuff penetration into hair. European patent application with laid open number EP 1 022 014 describes such compositions comprising anionic dyestuffs, solvents, as aid to enhance penetration of said dyestuffs, and a buffer solution to adjust the pH of the dyeing agent in the range from 2 to 6. For enhancing penetration of dyestuffs, solvents are used such as ethanol, benzyl alcohol, propylene carbonate, dipropylene glycol. Products are found on the professional hair dressing market applying this technology.

US 5,601,620, as well, discloses hair colouring agents with acid dyes, an organic solvent and at least one polysiloxane as a conditioner. The dyeing compositions disclosed here are having a pH in the range of 1.5 - 4.5.

Above two documents deal with the anionic dyes in acidic medium and they are silent on any colouring and/or lightening compositions at an alkaline pH and optionally comprising an oxidizing agent.

In an earlier European Patent application from our company with the application number 04 001 799.8, hair colouring composition is disclosed based on only anionic acidic direct dyes at alkaline medium. In that application, the possibility of addition of cationic dyestuff is disclosed to be only at minor quantities, preferably is not advised.

EP 810 851, on contrary to the above two publications, discloses hair colouring and lightening compositions comprising a cationic direct dye and an oxidizing agent. This documents is totally silent on any composition comprising anionic dyes.

WO 03/076518 discloses a process for converting sparingly soluble salts of cationic organic compounds and inorganic acids into more readily soluble salts of organic acids. The cationic compounds disclosed include cationic dyestuff and anionic dyestuffs are suggested for the above process. However, it was not shown and suggested to combine anionic dyes and cationic dyes in order to achieve more durable hair colour.

In practice, further development is obviously desired by professional hair dressing practitioners and also by end consumers in order to achieve highly brilliant and long lasting colorations. Additionally, colour resistance against washing and environmental influences is highly desirable. Furthermore, partial colourations in form of streaks has become popular and products are desired showing optimal colouring and at the same time optimal lightening performance for colourful hair appearance.

This invention starts with the above mentioned problems and discloses primarily a hair colouring and secondarily a lightening composition with excellent colouring and highlighting effects together with excellent stability against washing (shampooing) and environmental influences.

Inventors of the present invention have surprisingly selected from Acid Red 52, DC Red 33, DC Orange 4, and DC Yellow 10 found out that hair colouring compositions on aqueous basis comprising at least one direct acting anionic dyestuff and at least one direct action cationic dyestuff show excellent colouring ability and excellently stable against washing (shampooing) and environmental influences. Additionally, colouring compositions of the present invention are excellently suitable for lightening and colouring purposes when mixed with an oxidizing agent prior to application.

According to the invention, suitable cationic dyestuffs are in principal those available on the market for cosmetic hair colouring applications. For this purpose, special reference is made to the PCT application WO 95/15144 of Ciba-Geigy AG.

The cationic dyestuff with the following chemical structure are especially, the most preferred one according to the present invention. 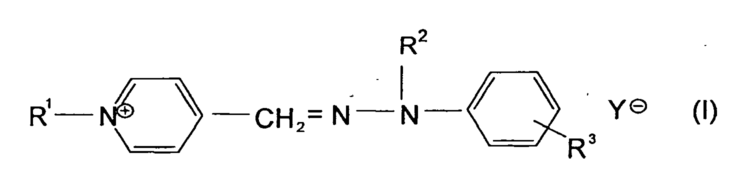 wherein R¹ and R² stand for hydrogen, a CH₃- or C₂H₅- group, R³ stands for hydrogen, -OCH₃ or -OC₂H₅ and.

The most preferred compound is the one according to the formula I, where R₁ and R₂, are methyl, R₃ is hydrogen and Y is methosulfate.

Additionally other cationic dyestuffs can as well be used in addition to the cationic dyestuffs mentioned above. Some examples to those are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

According to the invention, coloring composition comprises anionic dyes at a concentration of 0.1 to 7.5%, preferably 0.2 to 5%, more preferably 0.2 to 3% by weight calculated to total composition. Cationic dyestuffs are included into the compositions of the present invention at a concentration of 0.01 to 2.5%, preferably 0.05 to 2% and more preferably 0.05 to 1 % by weight calculated to total composition.

Interestingly it has as well been found out that the ratio of acidic dyes to cationic dyes plays an important role for achieving especially resistance to washing and environmental effects. Accordingly, the ratio of cationic dyestuffs to acidic dyestuffs by weight is in the range of 3:1 to 1:10, preferably 2: 1 to 1:7 and further more preferably 2:1 to 1:5.

Additionally, the coloring compositions of the present invention may comprise neutral dyes (HC dyes), so called nitro dyes for shading purposes. Concentration of those can typically be in the range from 0.01 to 2.5%, preferably 0.1 to 2% by weight calculated to total composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No. 15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

pH of the coloring compositions of the present invention varies between 5 and 12, preferably 6 - 11, more preferably 6.8 to 10. In the case that, the composition is used for highlighting (lightening) and coloring, the forgoing pH values refer to the pH of the dyeing composition before mixing with oxidizing agent. pH of the colouring composition is adjusted to the required pH by using triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

Colouring composition of present invention can comprise additionally in the base formulation fatty acids with 0 to 3 ethylenic bonds and with fatty acyl chain length of 12 to 22 C atom. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid. The most preferred fatty acid is oleic acid.

Colouring compositions according to the present invention can be in the form of emulsion, solution, dispersion and/or gel. Emulsion form is preferred.

In the case that the colouring composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 0.5 to 15% by weight, calculated to total composition.

Colouring compositions according to present invention comprises surfactants selected from anionic, nonionic, amphoteric (or zwiterionic) and/or cationic surfactants as emulsifier or solubilizer. Cationic surfactants are as well used as hair conditioners in the colouring composition.

Anionic surfactants suitable within the scope of the invention are in principal known from the cleansing compositions and are preferably present in an amount from 0.1, to about 10%, preferably 0.2 to 7.5% and most preferably 0.2 - 5% by weight, calculated to the total composition.

These are anionic surfactants of the sulfate, sulfonate, carboxylate and alkyl phosphate type, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₅-(C₂H₄O)ₙ-O-CH₂COOX,

wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 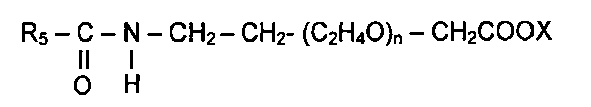 wherein R₅ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2^{nd} Ed.(1989, Hüthig Buchverlag), pp. 595-600 and pp. 683 to 691.

Further surfactants in the colouring compositions according to the invention are nonionic surfactants alone or in admixture with anionic surfactants at a weight ratio of 3:1 to 1:3.

These are described as well in Schrader, I.c., on pages 600-601 and pp. 694-695.

Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide.

Further nonionic surfactants suited are alkyl polyglucosides of the general formula

R₆-O-(R₃O)ₙ-Zₓ,

wherein R₆ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

As further surfactant component, the colouring compositions according to the invention can also contain amphoteric or zwitterionic surfactants, for example in an amount from about 0.5 % to about 5 %, preferably from about 1 % to about 2.5 % by weight, calculated to the total composition.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structures 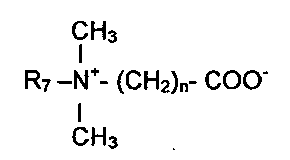 and 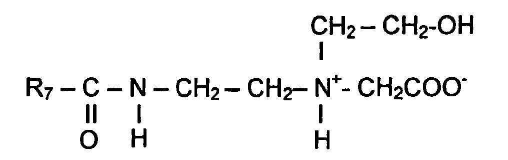 wherein R₇ is a C₈-C₁₈-alkyl group and n is 1 to 3; sulfobetaines of the structure 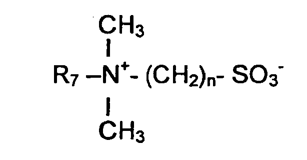 wherein R₇ and n are same as above;
and amidoalkyl betaines of the structure 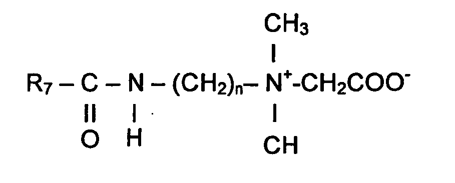 wherein R₇ and n are same as above.

Colouring composition can contain cationic surfactans as emulsifier, solubilizer and/or conditioning ingredients according to the formula, but not limited to. 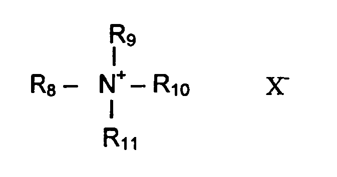 where R₈ is a saturated or unsaturated, branched or non-branched alkyl chain with 8-22 C atoms or

R₁₂ CO NH (CH₂)ₙ

where R₁₂ is saturated or unsaturated, branched or non-branched alkyl chain with 7-21 C atoms and n has typical value of 1 - 4 or

R₁₃ CO O (CH₂)ₙ

where R₁₃ is saturated or unsaturated, branched or non-branched alkyl chain with 7-21 C atoms and n has typical value of 1 - 4, and R₉ is H or unsaturated or saturated, branched or non-branched alkyl chain with 1 - 22 C atoms or

R₁₂CO NH (CH₂)ₙ

or

R₁₃ CO O (CH₂)ₙ

where R₁₂, R₁₃ and n are same as above.

R₁₀ and R₁₁ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Colouring composition can also contain cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 6, Polyquatemium 7, Polyquatemium 10, Polyquatemium 11, Polyquatemium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

Typical concentration range for any of the cationic conditioners mentioned above can be 0.01 - 5% by weight, preferably 0.03 - 2.5% by weight and more preferably 0.05 - 1.5% by weight.

Hair dyeing composition of the present invention preferably comprise an organopolysiloxane wherein at least one silicium atom is linked to an alkylene group having a hetero-atom, in particular a nitrogen atom, with a poly-(N-acyl alkyleneimine) units of the formula 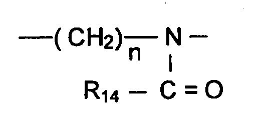 wherein n is a number from 1 to 5 and R₁₄ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 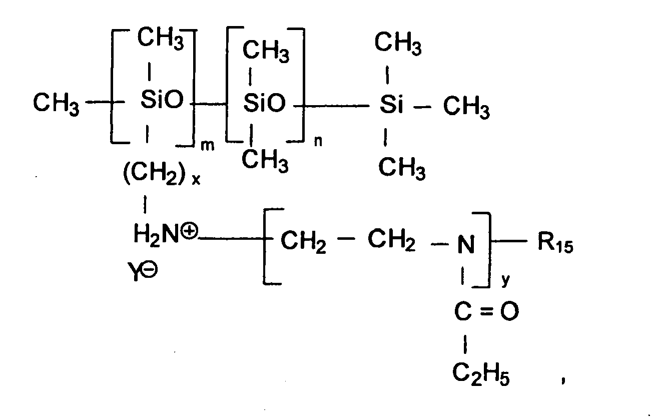 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Colouring compositions according to the present invention can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total composition.

The hair dyeing compositions according to the invention preferably contain thickening agents. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the colouring composition of this invention can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl - adipate, myristyl myristate and oleyl erucate.

Additional non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula X or XI, respectively,

R₁₆ CO (OCH₂CH₂)ₙ OH formula X

R₁₆ CO (OCH₂CH₂)ₙ O OC R₁₇ formula XI

where R₁₆ and R₁₇ are independent from each other saturated, unsaturated or branched or non-branched alkyl chain with 7 to 21 C atoms and n is typically 2 - 100.

Another preferred compound in the colouring composition is of ceramide type of compounds according to general formula 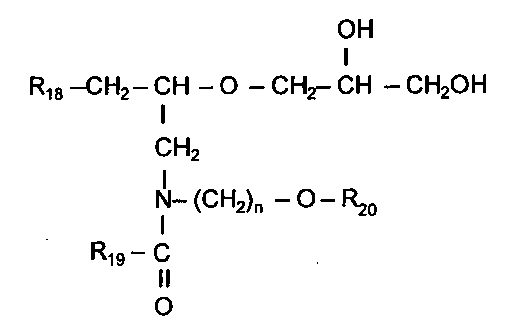 where R₁₈ and R₁₉ are independent from each other alkyl- or. alkenyl group mit 10 to 22 carbon atoms, R₂₀ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1 % by weight calculated to the total composition.

Colouring composition may as well contain UV filters of oil soluble, non-ionic, ones and/or as well those of water soluble and mainly of anionic character. Examples are Benzophenone-1 Benzophenone-2, Benzophenone-3, Benzophenone-7, Benzophenone-6, Benzophenone-8, octylmethoxy cinnamate, homosalat to those of oil soluble ones and Benzophenone-4, benzophenone-9 to those anionic water soluble ones. It should be noted that the other UV filters of oil and water soluble ones should as well be possible to combine.

Another preferred way of carrying out the present invention is that mixing colouring compositions with a composition comprising at least one oxidizing agent prior to application onto hair. By doing so, lightening (highlighting) and colouring is achieved at the same time. In another words, original hair colours is lightened and at the same time dyes, stable in the presence of oxidizing agent, are deposited onto hair so that new colour appearance is achieved. The oxidizing agents suitable are hydrogen peroxide, urea peroxide, melamin peroxide or perborate salts. The most preferred is hydrogen peroxide, which is used as a lotion containing 3 to 12% by weight, calculated to composition only comprising hydrogen peroxide.

The new composition as a result of mixing colouring and oxidizing composition allows achieving simultaneous lightening and colorations. The mixing ratio of the colouring composition and oxidizing composition should be in the range of 4:1 to 1:4, by weight, preferably 2:1 to 1:3 by weight.

Colouring and highlighting with compositions of the present invention can be carried out in different ways of processing.

One of the processes is that colouring composition is mixed with an oxidizing composition and applied onto hair and after a processing time, depending on the wished lightening and as well colour tone, processed for 5 to 45 min and rinsed off from hair.

Another way of carrying out highlighting and colouring is that of two step application. In the first step, composition comprising at least one oxidizing agent is applied onto hair and left on the hair for 5 to 45 min and without rinsing off, the colouring agent is applied onto hair as a second step and after leaving onto hair additional 5 to 45 min the mixed compositions are rinsed of from hair.

In the above mentioned two step colouring and highlighting process, between the application of first and second agents, the hair can be washed with water and optionally hair can be dried.

In the lightening and colouring process using the colouring composition of the present invention, the lightening can as well be carried out with the composition known as bleaching agents. For such a process suitable bleaching composition is for example the one disclosed in a European Patent No 560 088. Preferred way of carrying out lightening an colouring using bleaching agents is two step process as mentioned above.

Another way of carrying out the invention is that addition of oxidation dyestuffs precursors (developing substances) and coupling substances into the colouring compositions of the present invention. Those oxidative dyes can as well be mixed into the colouring composition prior to application onto hair. It is possible to incorporate developing substances known **per se.** In the case of oxidation dyes are present in the compositions, colouring is than carried out in the presence of oxidizing agent, i.e. oxidative dye containing colouring composition is mixed with oxidizing agent prior to application. Special mention is made of p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy-pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

The total concentration of the developing substances customarily ranges between about 0.05 % and 5 %, preferably 0.1 % and 4 %, in particular 0.1 % to 3 % by weight, calculated to the total hair dyeing composition (excluding the oxidation agent), whereby these figures are always related to the proportion of free base.

The composition according to the present invention can contain coupling substances, which can be selected from 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

The composition of the present invention can contain additional ingredients such as preservatives, chelating agents, fragrance and substances customarily used in cosmetic colouring compositions.

The viscosity of the compositions according to the invention preferably ranges from 1,000 to 50,000, in particular 1,000 to 30,000, especially 1,000 to 20,000 mPa.s, measured at 20°C with a Brookfield rotation viscosimeter, with spindle 5 at 5 rpm.

The invention is illustrated with the following examples, but not limited to.

**Base formulations**

| | % by weight | % by weight |
|---|---|---|
| | I | II |
| Stearamide MEA | - | 1.50 |
| Cocamide MEA | 4.00 | 2.00 |
| Cetearyl alcohol | 10.00 | 8.00 |
| Tegin P | 1.40 | 1.40 |
| Propylene Glycol | 2.40 | 1.60 |
| Oleic acid | 3.00 | 3.00 |
| Ammonium chloride | 0.50 | 0.50 |
| Tetrasodium EDTA | 0.20 | 0.20 |
| Sodium lauryl sulfate | 1.50 | - |
| Sodium cetearyl sulfate | - | 1.20 |
| Organopolysiloxane A1 of EP 640 643 | 0.20 | 0.20 |
| Ceramide according to formula wherein | | |
| R₁₈ and R₁₉ are C16 and R₂₀ is ethyl | - | 0.20 |
| Water | to 100 | to 100 |

The dyestuffs given in the following as for various color directions are added to the base compositions either I or II. Water amount is reduced in the base formula corresponding to the amount of dyes present in the formulations.

### Dyestuff composition examples:

**Table I**

| | % by weight | | |
|---|---|---|---|
| | Yellow | Shiny orange red | Orange |
| Acid Red 52 | - | 1.50 | - |
| DC Yellow10 | 1.50 | - | - |
| DC Red 33 | - | - | - |
| DC Orange 4 | - | - | 1.50 |
| Dyestuff of formula 1 | 0.50 | 0.50 | 0.50 |
| R₁=R₂= methyl and R₃=H and | | | |
| Y is methosulfate | | | |
| Base formula | I or II | I or II | I or II |

**Table II**

| | % by weight |
|---|---|
| | Orange (reddish) |
| Acid Red 52 | - |
| DC Yellow 10 | - |
| DC Red 33 | 1.50 |
| DC Orange 4 | - |
| Dyestuff of formula I | 0.50 |
| R₁=R₂ = methyl and R₃=H and | |
| Y is methosulfate | |
| Base formula | I or II |

The coloring compositions so obtained show excellent dyeing performance when applied as they are without premixing with any other composition.

In addition, the coloring compositions are mixed with a solution comprising 12% by weight hydrogen peroxide at a ratio of 1:2 (coloring mass: peroxide solution) and applied onto hair, in all cases, excellent highlighting and colouring effects are observed.

In order show color fastness against washing, an intensive yellow colored hair tress with a composition comprising D&C Yellow 10 and cationic yellow dye according to formula I with R₁=R₂=methyl and R₃=H and Y is methosulfate as presented above, was washed 10 times under usual hair wash conditions with a commercial shampoo composition designed for colored hair of the trade mark Goldwell Definition, and color of the tresses (L, a and b values) were measured before and after shampooing optically with a laboratory equipment and color differences were calculated with the well known equation to obtain ΔE values and color intensity differences (ΔL) were obtained from measured L values. The results are presented in Tablelll below. The test was as well carried out with compositions comprising only the cationic dyestuff of formula I with R₁=R₂= methyl and R₃=H and Y is methosulfate, at a concentration as incorporated into the inventive coloring composition and at a concentration equal to the total dyestuff content of the inventive composition (sum of concentrations anionic and cationic dyestuffs).

**Table III: Results of durability test against wash - shampooing**

| | ΔE | ΔL |
|---|---|---|
| Inventive composition | | |
| 0.5% cationic and 1.5% | 3.7 | 3.5 |
| anionic dyestuffs | | |
| Comparative composition | | |
| with 0.5% cationic dye | 11.7 | 0.9 |
| Comparative composition | | |
| with 2% cationic dye | 4.6 | 2.2 |

The results in the table should be understood as the lower the value, the better the colourfastness against washing. Thus, the most stable color is obtained with the inventive composition.

## Claims

1. Coloring composition for keratin fibers, especially for hair **characterized in that** it comprises at least one acidic direct dye selected from Acid Red 52, DC Yellow 10, DC Orange 4 and DC Yellow 10 and at least one cationic direct dye selected from compounds presented with general formula 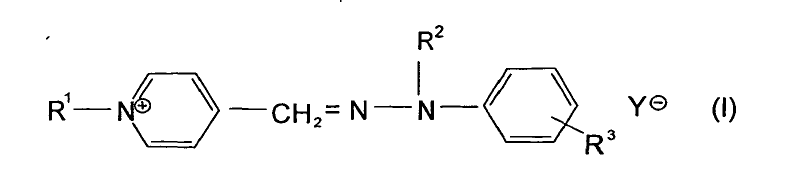 wherein R¹ and R² stand for hydrogen, a CH₃- or C₂H₅- group, R³ stands for hydrogen, -OCH₃ or -OC₂H₅, and Y⁻ is chloride, bromide, methosulfate, and having a pH between 5 and 12.

2. Composition according to claim 1 **characterized in that** cationic dyestuff preferred is according to formula I wherein R¹ and R² are methyl, R³ is hydrogen and Y⁻ is methosulfate.

3. Composition according to any of the preceding claims **characterized in that** pH of the composition is between 6.8 and 10.

4. Composition according to any of the preceding claims **characterized in that** it comprises at least one cationic dyestuff and at least one acidic dyestuff at a weight ratio of 3:1 to 1:10, preferably 2:1 to 1:7.

5. Composition according to any of the preceding claims **characterized in that** it comprises anionic direct dye at a concentration of 0.1 - 7.5% by weight, calculated to the total composition.

6. Composition according to any of the preceding claims **characterized in that** it comprises cationic direct dye at a concentration of 0.01 - 2.5% by weight, calculated to the total composition.

7. Composition according to any of the preceding claims **characterized in that** it comprises HC dyes in addition to anionic and cationic direct dyes.

8. Composition according to any of the preceding claims **characterized in that** it comprises an additional cationic direct dye.

9. composition according to any of the preceding claims **characterized in that** it further comprises saturated and/or unsaturated fatty acids with 0 to 3 ethylenic bonds and a fatty acyl chain length of 12 to 22 C atoms.

10. Composition according to claim 9 **characterized in that** the fatty acid is oleic acid.

11. Composition according to any of the preceding claims **characterized in that** it further comprises fatty alcohol and one or more surfactants selected from anionic, nonionic, amphoteric and/or cationic surfactants.

12. Composition according to claim 11 **characterized in that** anionic surfactants are selected from alkyl sulfates and alkyl ether sulfates, nonionic surfactants are selected from fatty acids mono or diethanolamides and fatty alcohols are with a fatty acyl chain length of 14 to 22 C atoms.

13. Composition according to any of the preceding claims **characterized in that** it comprises organosiloxane polymer according to formula 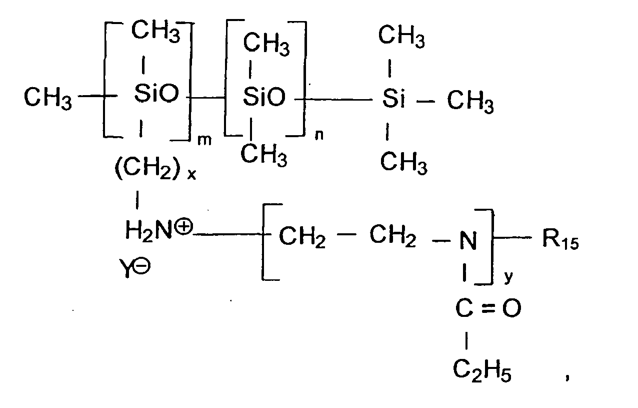 wherein m and n each are numbers from 20 to 10,000, x is a number between 1 and 5, and y is a number from 5 to 30, R₁₅ is a C₁-C₁₂-alkyl or aryl group, in particular a methyl, ethyl or benzyl group, and Y⁻ is an anion.

14. Composition according to any of the preceding claims **characterized in that** it comprises additionally ceramide type compound according to the formula 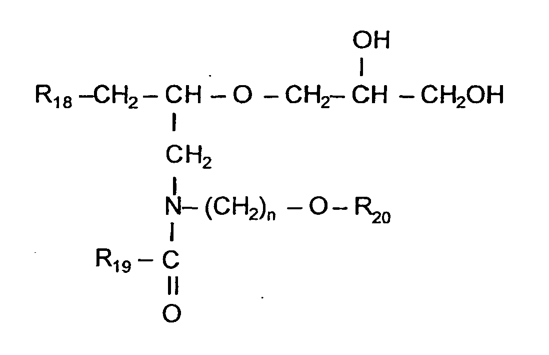 where R₁₈ and R₁₉ are independent from each other alkyl- or. alkenyl group mit 10 to 22 carbon atoms, R₂₀ is methyl, ethyl, n-propyl or isopropyl group and n is a number between 1 to 6, preferably 2 or 3.

15. composition according to any of the preceding claims **characterized in that** it comprises additionally organic solvents.

16. Composition according to any of the preceding claims **characterized in that** it comprises additionally UV absorbers selected from anionic water soluble ones and/or nonionic oil soluble ones.

17. Composition according to any of the preceding claims **characterized in that** it is mixed with a composition comprising at least one oxidizing agent prior to application onto hair at a weight ratio of 4:1 to 1:4.

18. composition according claim 17 **characterized in that** oxidizing agent is hydrogen peroxide at a concentration of 3 to 12% by weight calculated to total composition.

19. Composition according to claim 17 **characterized in that** it comprises additionally oxidative dyes precursors and coupling substances.

20. Use of composition according to any of the preceding claims for colouring hair.

21. Use of composition according to claims 17 and 18 for colouring and lightening hair.

22. Process for colouring and lightening hair **characterised in that** a composition according to claims 17 and 18 is applied onto hair and left 5 to 45 min and rinsed off from hair.

23. Process for colouring and lightening hair **characterised in that** an oxidizing composition comprising at least one oxidizing agent is applied onto dry hair and left for 5 to 45 min on hair and without rinsing it off from hair a colouring composition according to claims 1-16 is applied onto hair and left further for 5 to 45 min on hair and rinsed off from hair.

24. Process according to claim 23 **characterised in that** before application of colouring composition oxidizing agent is rinsed off from hair and hair dried and subsequently a colouring composition according to claims 1-16 is applied onto hair and left further for 5 to 45 min on hair and rinsed off from hair.

## Patentansprüche

1. Färbemittel für Keratinfasern, insbesondere für das Haar, **dadurch gekennzeichnet, dass** es mindestens einen sauren, direktziehenden Farbstoff, ausgewählt aus Acid Red 52, DC Yellow 10, DC Red 33, DC Orange 4 und DC Yellow 10, und mindestens einen kationischen, direktziehenden Farbstoff, ausgewählt aus Verbindungen, dargestellt mit der allgemeinen Formel 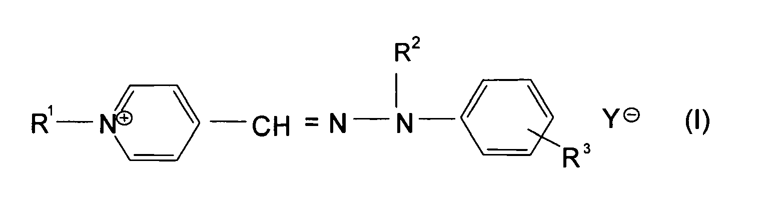 worin R¹ und R² für Wasserstoff , eine CH₃- oder C₂H₅- Gruppe steht, R³ für Wasserstoff, -OCH₃ oder -OC₂H₅ steht, und Y⁻ ein Chlorid, Bromid, oder Methosulfat ist,
enthält, und einen pH-Wert zwischen 5 und 12 hat.

2. Mittel nach Anspruch 1, **dadurch gekennzeichnet, dass** der kationische Farbstoff nach Formel 1 bevorzugt wird, worin R₁ und R₂ Methyl ist, R₃ Wasserstoff und Y⁻ ein Methosulfat.

3. Mittel nach einem der vorhergehenden Ansprüche, **dadurch gekennzeichnet, dass** der pH-Wert des Mittels zwischen 6,8 und 10 liegt.

4. Mittel nach einem der vorhergehenden Ansprüche, **dadurch gekennzeichnet, dass** es mindestens einen kationischen Farbstoff und mindestens einen sauren Farbstoff in einem Gewichtsverhältnis von 3:1 bis 1:10, vorzugsweise 2:1 bis 1:7, enthält.

5. Mittel nach einem der vorhergehenden Ansprüche, **dadurch gekennzeichnet, dass** es anionischen, direktziehenden Farbstoff in einer Menge von 0,1 bis 7,5 Gew. %, berechnet auf die Gesamtzusammensetzung, enthält.

6. Mittel nach einem der vorhergehenden Ansprüche, **dadurch gekennzeichnet, dass** es kationischen, direktziehenden Farbstoff in einer Menge von 0,01 bis 2,5 Gew. %, berechnet auf die Gesamtzusammensetzung, enthält.

7. Mittel nach einem der vorhergehenden Ansprüche, **dadurch gekennzeichnet, dass** es zusätzlich zu den anionischen und kationischen direktziehenden Farbstoffen auch HC-Farbstoffe enthält.

8. Mittel nach einem der vorhergehenden Ansprüche, **dadurch gekennzeichnet, dass** es einen zusätzlichen kationischen, direktziehenden Farbstoff enthält.

9. Mittel nach einem der vorhergehenden Ansprüche, **dadurch gekennzeichnet, dass** es weiterhin gesättigte und/oder ungesättigte Fettsäuren mit 0 bis 3 Ethylenverbindungen und einer Fettacyl- Kettenlänge von 12 bis 22 C-Atomen enthält.

10. Mittel nach Anspruch 9, **dadurch gekennzeichnet, dass** die Fettsäure, Ölsäure ist.

11. Mittel nach einem der vorhergehenden Ansprüche, **dadurch gekennzeichnet, dass** es weiterhin Fettalkohol und ein oder mehrere Tenside, ausgewählt aus anionischen, nichtionischen, amphoterischen und/oder kationischen Tensiden, enthält.

12. Mittel nach Anspruch 11, **dadurch gekennzeichnet, dass** die anionischen Tenside aus Alkylsulfaten und Alkylethersulfaten, die nichtionischen Tenside aus Fettsäuremono- oder diethanolamiden ausgewählt wurden, und die Fettalkohole eine Fettacyl- Kettenlänge von 14 bis 22 C-Atomen haben.

13. Mittel nach einem der vorhergehenden Ansprüche, **dadurch gekennzeichnet, dass** es Organosiloxan-Polymere entsprechend der Formel 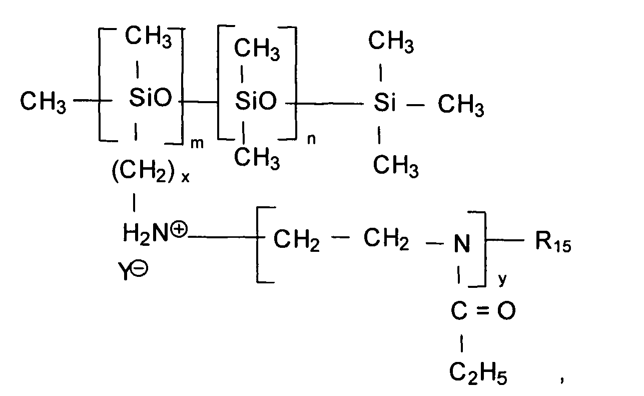 enthält,
worin m und n jeweils Zahlen von 20 bis 10.000 sind, x eine Zahl zwischen 1 und 5, und y eine Zahl von 5 bis 30 ist, R₁₅ ist eine C₁-C₁₂-Alkyl oder Aryl-Gruppe, insbesondere eine Methyl-, Ethyl- oder Benzyl- Gruppe, und Y- ein Anion.

14. Mittel nach einem der vorhergehenden Ansprüche, **dadurch gekennzeichnet, dass** es zusätzlich Ceramid-Verbindungen entsprechend der Formel 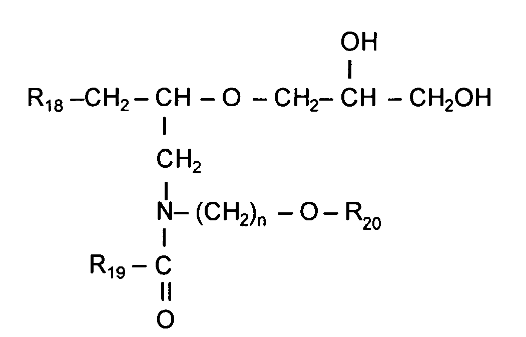 enthält,
wobei R₁₈ und R₁₉, unabhängig voneinander, Alkyl- oder Alkenyl-Gruppen mit 10 bis 22 Kohlenstoffatomen sind, R₂₀ ist eine Methyl-, Ethyl-, n-Propyl- oder Isopropyl- Gruppe, und n eine Zahl zwischen 1 bis 6, vorzugsweise 2 oder 3.

15. Mittel nach einem der vorhergehenden Ansprüche, **dadurch gekennzeichnet, dass** es zusätzlich organische Lösungsmittel enthält.

16. Mittel nach einem der vorhergehenden Ansprüche, **dadurch gekennzeichnet, dass** es zusätzlich UV-Absorber, ausgewählt aus anionischen, wasserlöslichen und /oder nichtionischen öllöslichen, enthält.

17. Mittel nach einem der vorhergehenden Ansprüchen, **dadurch gekennzeichnet, dass** es vor dem Auftragen auf das Haar, mit einem Mittel das mindestens einen oxidierenden Wirkstoff enthält, in einem Gewichtsverhältnis von 4:1 bis 1:4 gemischt wird.

18. Mittel nach Anspruch 17, **dadurch gekennzeichnet, dass** der oxidierende Wirkstoff Wasserstoffperoxid in einer Konzentration von 3 bis 12 Gew. %, berechnet auf die Gesamtzusammensetzung, ist.

19. Mittel nach Anspruch 17, **dadurch gekennzeichnet, dass** es zusätzlich Oxidationsfarbstoffvorprodukte und Kupplersubstanzen enthält.

20. Verwendung eines Mittels nach einem der vorhergehenden Ansprüche zum Färben von Haaren.

21. Verwendung eines Mittels nach den Ansprüchen 17 und 18 zum Färben und Aufhellen von Haaren.

22. Verfahren zum Färben und Aufhellen von Haaren, **dadurch gekennzeichnet, dass** ein Mittel nach den Ansprüchen17 und 18 auf das Haar aufgetragen, 5 bis 45 Minuten darauf belassen und dann aus dem Haar ausgespült wird.

23. Verfahren zum Färben und Aufhellen von Haaren, **dadurch gekennzeichnet, dass** eine oxidierende Zusammensetzung, enthaltend mindestens einen oxidierenden Wirkstoff, auf trockenes Haar aufgetragen, und für 5 bis 45 Minuten darauf belassen wird, und, ohne sie auszuspülen, ein Färbemittel nach Anspruch 1 bis 16 auf das Haar aufgetragen wird, weitere 5 bis 45 auf dem Haar bleibt und dann vom Haar abgespült wird.

24. Verfahren nach Anspruch 23, **dadurch gekennzeichnet, dass** vor der Applikation des Färbemittels der oxidierende Wirkstoff aus dem Haar ausgespült, und das Haar getrocknet wird und anschließend ein Färbemittel nach den Ansprüchen 1 bis 16 auf das Haar aufgetragen, und weitere 5 bis 45 Minuten auf dem Haar belassen, und dann ausgespült wird.

## Revendications

1. Composition de coloration pour des fibres de kératine, notamment pour des cheveux **caractérisée en ce qu'**elle comprend au moins un colorant direct acide choisi parmi Acid Red 52, DC Yellow 10, DC Red 33, DC Orange 4 et DC Yellow 10 et au moins un colorant direct cationique choisi parmi les composés présentés par la formule générale 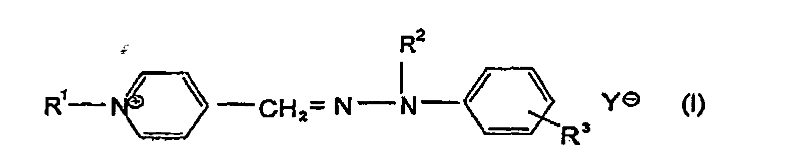 dans laquelle R¹ et R² représentent un atome d'hydrogène, un groupe CH₃ ou C₂H₅, R³ représente un atome d'hydrogène, -OCH₃ ou -OC₂H₅, et Y⁻ représente un chlorure, un bromure, un méthosulfate, et qui possède un pH compris entre 5 et 12.

2. Composition selon la revendication 1, **caractérisée en ce que** la matière colorante cationique préférée est selon la formule I dans laquelle R¹ et R² représentent un groupe méthyle, R³ représente un atome d'hydrogène et Y⁻ représente un méthosulfate.

3. Composition selon l'une quelconque des revendications précédentes, **caractérisée en ce que** le pH de la composition est compris entre 6,8 et 10.

4. Composition selon l'une quelconque des revendications précédentes, **caractérisée en ce qu'**elle comprend au moins une matière colorante cationique et au moins une matière colorante acide à un rapport en poids de 3:1 à 1:10, de préférence de 2:1 à 1:7.

5. Composition selon l'une quelconque des revendications précédentes, **caractérisée en ce qu'**elle comprend un colorant direct anionique à une concentration de 0,1 à 7,5 % en poids, calculée par rapport à la composition totale.

6. Composition selon l'une quelconque des revendications précédentes, **caractérisée en ce qu'**elle comprend un colorant direct cationique à une concentration de 0,01 à 2,5 % en poids, calculée par rapport à la composition totale.

7. Composition selon l'une quelconque des revendications précédentes, **caractérisée en ce qu'**elle comprend des colorants HC en plus des colorants directs anioniques et cationiques.

8. Composition selon l'une quelconque des revendications précédentes, **caractérisée en ce qu'**elle comprend un colorant direct cationique supplémentaire.

9. Composition selon l'une quelconque des revendications précédentes, **caractérisée en ce qu'**elle comprend de plus des acides gras saturés et/ou insaturés avec 0 à 3 liaisons éthyléniques et une longueur de chaîne d'acyle gras de 12 à 22 atomes de C.

10. Composition selon la revendication 9, **caractérisée en ce que** l'acide gras est un acide oléique.

11. Composition selon l'une quelconque des revendications précédentes, **caractérisée en ce qu'**elle comprend de plus un alcool gras et un ou plusieurs tensioactifs choisis parmi des tensioactifs anioniques, non ioniques, amphotères et/ou cationiques.

12. Composition selon la revendication 11, **caractérisée en ce que** les tensioactifs anioniques sont choisis parmi des alkylsulfates et des alkyléthersulfates, les tensioactifs non ioniques sont choisis parmi des mono- ou diéthanolamides d'acides gras et les alcools gras se trouvent avec une longueur de chaîne d'acyle gras de 14 à 22 atomes de C.

13. Composition selon l'une quelconque des revendications précédentes, **caractérisée en ce qu'**elle comprend un polymère d'organosiloxane selon la formule 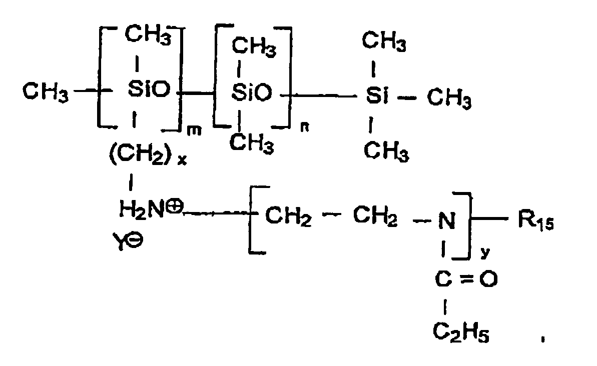 dans laquelle m et n représentent chacun des nombres de 20 à 10 000, x représente un nombre compris entre 1 et 5, et y représente un nombre de 5 à 30, R₁₅ représente un groupe alkyle en C₁ à C₁₂ ou aryle, en particulier un groupe méthyle, éthyle ou benzyle, et Y⁻ représente un anion.

14. Composition selon l'une quelconque des revendications précédentes, **caractérisée en ce qu'**elle comprend en outre un composé de type céramide selon la formule 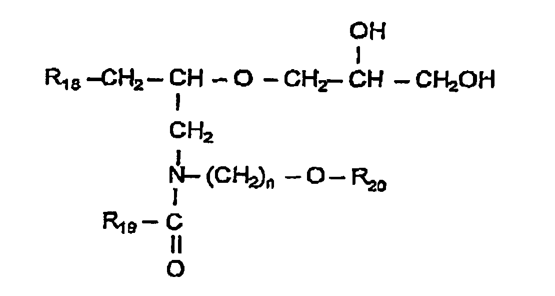 dans laquelle R₁₈ et R₁₉ représentent indépendamment l'un de l'autre un groupe alkyle ou alcényle avec 10 à 22 atomes de carbone, R₂₀ représente un groupe méthyle, éthyle, n-propyle ou isopropyle et n représente un nombre compris entre 1 et 6, de préférence 2 ou 3.

15. Composition selon l'une quelconque des revendications précédentes, **caractérisée en ce qu'**elle comprend en outre des solvants organiques.

16. Composition selon l'une quelconque des revendications précédentes, **caractérisée en ce qu'**elle comprend en outre des absorbants d'U.V. choisis parmi ceux anioniques solubles dans l'eau et/ou ceux non ioniques solubles dans l'huile.

17. Composition selon l'une quelconque des revendications précédentes, **caractérisée en ce qu'**on la mélange avec une composition comprenant au moins un agent oxydant avant une application sur les cheveux à un rapport en poids de 4:1 à 1:4.

18. Composition selon la revendication 17, **caractérisée en ce que** l'agent oxydant est du peroxyde d'hydrogène à une concentration de 3 à 12 % en poids calculée par rapport à la composition totale.

19. Composition selon la revendication 17, **caractérisée en ce qu'**elle comprend en outre des précurseurs de colorants par oxydation et des substances de couplage.

20. Utilisation de composition selon l'une quelconque des revendications précédentes pour la coloration de cheveux.

21. Utilisation de composition selon les revendications 17 et 18 pour la coloration et l'éclaircissement de cheveux.

22. Procédé pour la coloration et l'éclaircissement de cheveux, **caractérisé en ce que** l'on applique sur les cheveux une composition selon les revendications 17 et 18, et on la laisse 5 à 45 min puis on l'élimine des cheveux par rinçage.

23. Procédé pour la coloration et l'éclaircissement de cheveux, **caractérisé en ce que** l'on applique sur les cheveux secs une composition oxydante comprenant au moins un agent oxydant et on la laisse pendant 5 à 45 min sur les cheveux et sans l'éliminer des cheveux par rinçage, on applique sur les cheveux une composition de coloration selon les revendications 1 à 16, et on la laisse pendant encore 5 à 45 min sur les cheveux puis on l'élimine des cheveux par rinçage.

24. Procédé selon la revendication 23, **caractérisé en ce qu'**avant l'application de la composition de coloration, on élimine des cheveux par rinçage l'agent oxydant et l'on sèche les cheveux puis ultérieurement on applique sur les cheveux une composition de coloration selon les revendications 1 à 16, et on la laisse pendant encore 5 à 45 min sur les cheveux puis on l'élimine des cheveux par rinçage.
